# EUROPÄISCHE PATENTSCHRIFT

(11) **EP 2 288 394 B1**
(45) Veröffentlichungstag und Bekanntmachung des Hinweises auf die Patenterteilung: **02.08.2017**
(21) Anmeldenummer: 09757295.2
(22) Anmeldetag: 04.06.2009
(51) Int. Cl.: F16L 3/00, A61M 1/36

(54) **FALTBARER ORGANIZER FÜR BLUTSCHLAUCHELEMENTE**
FOLDABLE ORGANIZER FOR BLOOD LINE ELEMENTS
DISPOSITIF PLIABLE DE RANGEMENT FONCTIONNEL POUR DES ÉLÉMENTS FLEXIBLES D'ÉCOULEMENT SANGUIN

(30) Priorität: 05.06.2008 DE 102008026915
(43) Veröffentlichungstag der Anmeldung: 02.03.2011
(73) Patentinhaber: Fresenius Medical Care Deutschland GmbH, 61352 Bad Homburg (DE)
(72) Erfinder: KLEWINGHAUS, Jürgen, 61440 Oberursel (DE)
(74) Vertreter: Bobbert, Cornelius
(86) Internationale Anmeldenummer: PCT/EP2009/004009
(87) Internationale Veröffentlichungsnummer: WO 2009/146912

(56) Entgegenhaltungen:
- WO-A1-00/47266
- GB-A- 2 271 963
- GB-A- 2 334 250
- US-A- 4 512 466
- US-A- 5 379 906
- US-A1- 2003 194 262
- US-A1- 2003 220 598
- US-A1- 2006 086 634
- US-B1- 6 536 156

## Beschreibung

Die vorliegende Erfindung betrifft einen Organizer gemäß dem Anspruch 1.

Aus der Praxis sind Vorrichtungen für eine extrakorporale Blutbehandlung bekannt. Zur Behandlung des Patienten wird ein Blutschlauchsystem mit entsprechenden Anschlüssen der Behandlungsvorrichtung verbunden. Das Blutschlauchsystem wird zu seinem besseren Handling an einem Organizer angeordnet, und zwar derart, dass die entsprechenden Elemente des Schlauchsystems für ein Anschließen des Schlauchsystems mit der Vorrichtung bereits in die richtige Position gebracht sind. Ein Organizer, wie er beispielsweise aus der WO 2004/032782 A1 bekannt ist, weist einen flexiblen, flächigen Grundkörper auf, an welchem Halte- bzw. Befestigungseinrichtungen zum Aufnehmen von einzelnen Bestandteilen oder Elementen des Schlauchsystems vorgesehen sind. Der Organizer gibt den flexiblen Schlauchsystemelementen somit sowohl Halt als auch eine günstige Position relativ zur Vorrichtung.

Zur Aufnahme von Elementen des Schlauchssystems muss der Organizer eine gewisse Größe aufweisen. Seine Abmessungen können dabei zur besseren Handhabung des Organizers beim Transport oder Lagern durch ein Aufrollen oder -falten verringert werden. Hierdurch wird jedoch auf Elemente des Schlauchsystems eine unerwünschte Belastung ausgeübt, und es kann zum Knicken oder Deformieren von Blutschlauchelementen kommen. Letzteres kann zu plastischen Verformungen führen und hierdurch Nachteile mit sich bringen.

Weiterhin lässt sich ein aufgerollter oder gefalteter Organizer des Standes der Technik schlecht handhaben, da er sich mangels klarer, fester Begrenzungsstrukturen sowie mangelnder Formstabilität nur schwierig mit der Hand greifen oder beim Transport oder beim Lagern Platz sparend stapeln lässt. Beim aufgerollten oder gefalteten Organizer kann ferner die Lage der einzelnen Schlauchelemente zueinander verloren gehen.

Aus der WO 00/47266 A1 ist ein in sich geschlossener Organizer für einen extrakorporalen Blutkreislauf bekannt.

Aus der US 2006/0086634 A1 ist ein Organizer zur Verwendung in der Mikrochirurgie bekannt.

Aus der US 4,512,466 A ist ein Organizer für chirurgische Instrumente bekannt.

Aus der US 2003/0220598 A1 ist ein automatisiertes System für die peritoneale Dialyse bekannt.

Aufgabe der vorliegenden Erfindung ist es, einen weiteren Organizer anzugeben.

Die erfindungsgemäße Aufgabe wird gelöst durch die Merkmalskombination des Anspruchs 1.

So wird erfindungsgemäß ein Organizer vorgeschlagen, welcher wenigstens einen ersten, einen zweiten und einen dritten Abschnitt aufweist. Dabei ist der erste Abschnitt relativ zum zweiten Abschnitt bewegbar mit dem zweiten Abschnitt verbunden. Der zweite Abschnitt ist relativ zum dritten Abschnitt bewegbar mit dem dritten Abschnitt verbunden. Dabei kann der zweite Abschnitt - ebenso wie der erste und/ oder der dritte Abschnitt - mehrteilig ausgestaltet sein, wie dies beispielsweise in der angehängten Zeichnung zu erkennen ist. Der zweite Abschnitt kann dabei derart ausgestaltet sein, dass er einen definierten Abstand zwischen den mit ihm verbundenen Bereichen des ersten Abschnitts und des dritten Abschnitts zueinander sicherstellt. Abstandshalter zum Aufrechterhalten des Abstands zwischen dem ersten und dem dritten Abschnitt können vorteilhafterweise entbehrlich sein. Der zweite Abschnitt kann deren Funktion miterfüllen.

Unter einer relativen Bewegbarkeit wird erfindungsgemäß ein Schwenken, Falten, Biegen, Drehen oder dergleichen von zwei Abschnitten des Organizers zueinander verstanden. Dabei ist eine uneingeschränkte Bewegbarkeit jedoch nicht erforderlich. Die Bewegbarkeit zweier miteinander verbundener Abschnitte kann erfindungsgemäß durchaus aufgrund gegebener Materialsteifigkeit, durch zwischen den Abschnitten liegende Schlauchsystemelemente, und dergleichen beschränkt sein.

Unter einer Verbindung zwischen beispielsweise dem ersten Abschnitt und dem zweiten Abschnitt wird erfindungsgemäß jede Verbindung verstanden, mittels welcher ein Auseinanderfallen der jeweils miteinander verbundenen Abschnitte verhindert oder zu einem Verhindern beigetragen wird. Mittels der Verbindung kann eine Kraftübertragung zwischen den verbundenen Abschnitten möglich sein. Die Verbindung kann vorübergehender Art sein, sie kann jedoch auch die Lebensdauer des Organizers erreichen. Eine Verbindung im Sinne der vorliegenden Erfindung liegt auch dann vor, wenn miteinander verbundene Abschnitte mittels durchgehenden Materials, insbesondere einstückig, hergestellt sind. Die Verbindung kann flexibel ausgestaltet sein. Sie kann einen klar definierten, eckigen Übergang benachbarter Abschnitte zueinander erlauben oder sicherstellen. Die Verbindung kann einen variablen Abstand bspw. des ersten Abschnitts zum zweiten Abschnitt erlauben.

Weiterhin nimmt der Organizer aufgrund der äußeren Breite des zweiten Abschnitts im gefalteten Zustand eine Form an, an die eine Transportverpackung gut angepaßt werden kann. Dies wäre nicht der Fall, wenn der erste und der dritte Abschnitt, zwischen welchen sich die Komponenten des Blutschlauchsystems im zusammengeklappten Zustand des Organizers befinden, mit dem darin befindlichen Schlauchsystem direkt aufeinander liegen würden. Ein solch flexibles Paket hätte einen zu großen Bewegungsspielraum innerhalb einer, insbesondere starren, Verpackung. Das flexible Schlauchsystem allein besitzt kein reproduzierbares Dickenmaß und erschwert die Anpassung einer Verpackung.

Diese Eigenschaft der definierten Form des Organizers bzw. des definierten Abstands zumindest im Bereich des zweiten Abschnitts wird auch vorteilhaft genutzt beim Handling des Organizers durch den Benutzer. Dieser kann der Verpackung ein steifes, gefaltetes System entnehmen und dieses gut greifen, was erschwert wäre durch das Fehlen des klar definierten zweiten Abschnitts. Das dann "weich" verpackte Schlauchsystem ließe sich schlecht greifen.

So wird ein Organizer vorgeschlagen, welcher zwischen dem ersten Abschnitt und dem zweiten Abschnitt einen Sollabwinkelbereich oder eine Vorzugs-Biegelinie oder - Klapplinie oder -Faltlinie aufweist. Ein solcher Sollabwinkelbereich oder dergleichen entspricht jenem Bereich, welcher ähnlich einem Gelenk die definierte Relativbewegung zwischen dem ersten Abschnitt und dem zweiten Abschnitt ermöglicht.

Indem sich der Sollabwinkelbereich aufgrund seiner besonderen Ausgestaltung für eine Gelenkwirkung zwischen dem ersten und dem zweiten Abschnitt anbietet, können der erste Abschnitt und insbesondere der zweite Abschnitt bei einem Zusammenklappen oder Zusammenfalten des Organizers ihre Form unverändert beibehalten. Eine Relativbewegung zwischen dem ersten Abschnitt und dem zweiten Abschnitt erlaubt somit vorteilhaft ein Zusammenklappen oder -falten des Organizers, ohne dass dazu der erste und/ oder der zweite Abschnitt ebenfalls gebogen, gefaltet oder geklappt werden müssten. Der Sollabwinkelbereich kann dabei einteilig oder mehrteilig ausgestaltet sein. Er kann ausgestaltet sein, um eine Abwinkelbarkeit bis zu einem festgelegten Winkel, bspw. 90°, zwischen den benachbarten Abschnitten zu erlauben.

Ein solcher Sollabwinkelbereich kann auch zwischen dem zweiten Abschnitt und dem dritten Abschnitt des erfindungsgemäßen Organizers dieser Ausführungsform vorgesehen sein.

Der erfindungsgemäße Organizer weist einen Sollabwinkelbereich mit einer Materialdicke oder -stärke auf, welche geringer ist als eine Materialdicke oder -stärke des ersten Abschnitts und/ oder des zweiten Abschnitts.

Bei dieser Ausführung ist das Bewegen des ersten Abschnitts relativ zum zweiten Abschnitt durch eine einfache und kostengünstig vorzusehende Verringerung der Materialdicke im Bereich des Sollabwinkelbereichs ermöglicht oder zumindest begünstigt.

Vorteilhafte Weiterbildungen des erfindungsgemäßen Gegenstands sind jeweils Gegenstand der Unteransprüche.

In einer bevorzugten Ausfizhrungsform des erfindungsgemäßen Organizers ist der erste und/ oder der dritte Abschnitt durch wenigstens ein Filmscharnier mit dem zweiten Abschnitt verbunden. Das Filmscharnier kann hierbei eine oder mehrere, insbesondere zwei, Gelenkrillen aufweisen. Diese können voneinander beabstandet vorliegen. Das Filmscharnier erlaubt somit auf besonders zuverlässige Art ein definiertes Abknicken zweier mittels des Filmscharniers verbundener Abschnitte des Organizers.

Der zweite Abschnitt des Organizers kann in einer weiter bevorzugten Ausführungsform eine steifere Struktur als der erste Abschnitt oder der dritte Abschnitt aufweisen. Die steifere Struktur kann durch die Auswahl eines festeren Materials für den zweiten Abschnitt, durch ein dickeres Material im Bereich des zweiten Abschnitts in einer Richtung senkrecht zu einer Haupterstreckungsebene des zweiten Abschnitts oder durch das Vorsehen zusätzlicher, versteifend wirkender Elemente ausgestaltet sein.

Die steifere Struktur des zweiten Abschnitts dient vorteilhaft dazu, den durch den zweiten Abschnitt sichergestellten Abstand zwischen dem ersten Abschnitt und dem dritten Abschnitt - zumindest im Bereich ihrer Verbindung mit dem zweiten Abschnitt - im Gebrauch sicherzustellen. Der Grad der Steifigkeit des zweiten Abschnitts kann dabei derart durch konstruktive Maßnahmen bestimmt werden, dass der zweite Abschnitt seiner Abstandserhalterfunktion im normalen Gebrauch des Organizers gerecht wird, insbesondere ohne selber einzuknicken. Zudem ist durch die Steifigkeit des zweiten Abschnitts sichergestellt, dass in diesem Bereich verlaufende Blutschlauchelemente gegen ein Knicken gesichert sind.

Erfindungsgemäß weist der zweite Abschnitt wenigstens ein Halteelement zum reversiblen Aufnehmen von Blutschlauchteilen auf. Auch der erste und/oder der dritte Abschnitt weisen jeweils wenigstens ein Halteelement dieser oder anderer Art auf. Zu solchen Halteelementen zählen bspw. Schlauchhalter, Halter für venöse Klammern, etc.

Ein weiterer technischer Vorteil liegt darin, dass Schlauchteile, die am ersten Abschnitt befestigt sind, dauerhaft knickfrei mit Schlauchteilen des dritten Abschnitts verbunden werden können, indem sie im zweiten Abschnitt durch ein zusätzliches Halteelement gestützt oder geführt sind. Ist letzteres nicht der Fall, wie im Stand der Technik, so neigen Verbindungen, welche sich über den ersten und den dritten Abschnitt erstrecken, zum Knicken mit den hiermit verbundenen Nachteilen.

In einer wiederum weiter bevorzugten Ausführungsform weist der erfindungsgemäße Organizer einen einstückig hergestellten Grundkörper auf, welcher den ersten Abschnitt, den zweiten Abschnitt und den dritten Abschnitt aufweist. Dies erlaubt eine besonders einfache und kostengünstige Herstellung des erfindungsgemäßen Organizers.

In einer wiederum weiter bevorzugten Ausführungsform weist der Organizer wenigstens ein Polystyrol auf oder ist hieraus gefertigt. Die Herstellung des Organizers kann mittels Thermoformen erfolgen.

Die vorliegende Erfindung wird im Folgenden exemplarisch erläutert mit Bezug auf die beigefügte Figur. In der Figur gilt:
Fig. 1 zeigt einen faltbaren Organizer gemäß der vorliegenden Erfindung in einem gefalteten Zustand; und
Fig. 2 zeigt den Organizer aus Fig. 1 im geöffneten Zustand.

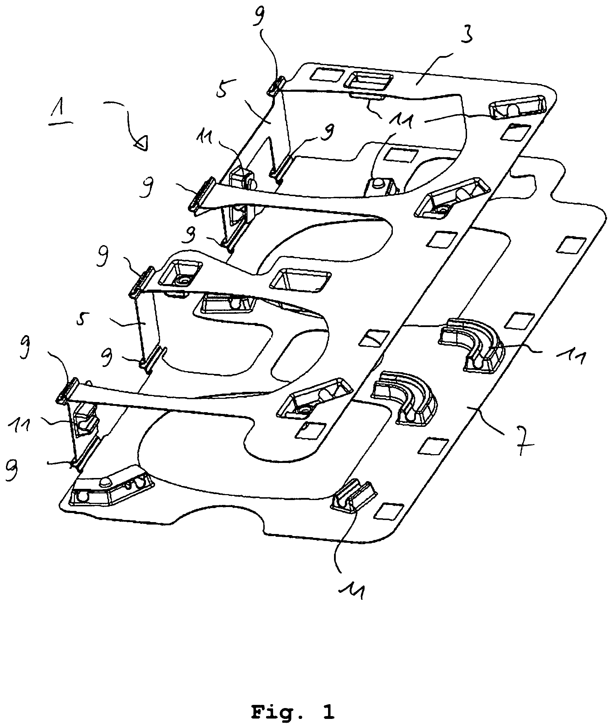

Fig. 1 zeigt einen faltbaren Organizer 1 mit einem ersten Abschnitt 3, einem zweiten Abschnitt 5 sowie einem dritten Abschnitt 7. Der erste Abschnitt 3 und der dritte Abschnitt 7 sind dabei mittels der Stützwirkung des zweiten Abschnitts 5 an ihren jeweils zum linken Bildrand hin gelegenen Enden voneinander beabstandet und zugleich miteinander verbunden. Die Verbindung zwischen erstem Abschnitt 3 und zweitem Abschnitt 5 erfolgt mittels mehrerer Sollabwinkelbereiche 9. Der zweite Abschnitt 5 ist mit dem dritten Abschnitt 7 ebenfalls mittels Sollabwinkelbereiche 9 verbunden.

Fig. 1 ist zu entnehmen, dass jeder der drei Abschnitte 3, 5 und 7 jeweils mehrere Halteelemente 11 zum Befestigen von in Fig. 1 nicht gezeigten Blutschlauchsystemelementen aufweist. Die Halteelemente 11 des zweiten Abschnitts 5 bewirken aufgrund ihrer Verbindung mit dem zweiten Abschnitt 5 bereits eine Versteifung des zweiten Abschnitts 5. Ferner dienen auch in die Halteelemente 11 des zweiten Abschnitts 5 aufgenommene - in Fig. 1 nicht gezeigte - Blutschlauchsystemelemente einer weiteren Versteifung des zweiten Abschnitts 5.

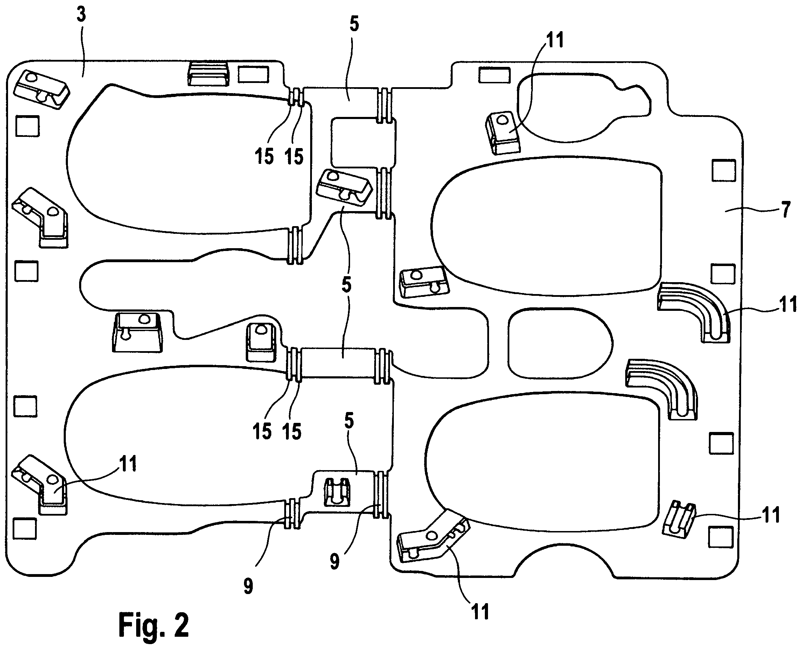

Fig. 2 zeigt den Organizer 1 aus Fig. 1 in einem ausgebreiteten oder aufgeklappten Zustand. Zu erkennen ist, dass der erste Abschnitt 3, der zweite Abschnitt 5 und der dritte Abschnitt 7 einstückig aus einem Grundkörper 13 hergestellt sind.

Die Sollabwinkelbereiche 9 weisen jeweils ein Filmscharnier mit exemplarisch je zwei Gelenkrillen 15 auf. Sie können dergestalt sein, dass sie ein Abknicken der Abschnitte zueinander nicht über einen vorbestimmten Winkel hinaus erlauben, was vorteilhaft zu erhöhter Festigkeit des Organizers beitragen kann.

## Patentansprüche

1. Organizer (1) mit wenigstens einem ersten, einem zweiten und einem dritten Abschnitt, wobei der erste Abschnitt (3) relativ zum zweiten Abschnitt (5) bewegbar mit dem zweiten Abschnitt (5) verbunden ist, und wobei der zweite Abschnitt (5) relativ zum dritten Abschnitt (7) bewegbar mit dem dritten Abschnitt (7) verbunden ist, wobei zwischen dem ersten Abschnitt (3) und dem zweiten Abschnitt (5) und/ oder zwischen dem zweiten Abschnitt (5) und dem dritten Abschnitt (7) wenigstens ein Sollabwinkelbereich (9) ausgestaltet ist, wobei der Sollabwinkelbereich (9) eine Materialdicke aufweist, welche geringer ist als eine Materialdicke des ersten Abschnitts (3) und/ oder des zweiten Abschnitts (5) und/ oder des dritten Abschnitts (7), **dadurch gekennzeichnet, dass** der zweite Abschnitt (5) sowie der erste Abschnitt (3) und/oder der dritte Abschnitt (7) jeweils wenigstens ein Halteelement (11) zum reversiblen Aufnehmen von Blutschlauchsystemelementen oder Blutschlauchteilen umfasst.

2. Organizer (1) nach Anspruch 1, wobei der erste Abschnitt (3) und/ oder der dritte Abschnitt (7) mit dem zweiten Abschnitt (5) durch wenigstens ein Filmscharnier verbunden sind.

3. Organizer (1) nach einem der vorangegangenen Ansprüche, wobei der dritte Abschnitt (7) mit dem ersten Abschnitt (3) nur über den zweiten Abschnitt (5) verbunden ist.

4. Organizer (1) nach einem der vorangegangenen Ansprüche, wobei der zweite Abschnitt (5) mehrteilig ausgestaltet ist.

5. Organizer (1) nach einem der vorangegangenen Ansprüche, wobei der zweite Abschnitt (5) eine steifere Struktur als der erste Abschnitt (3) und/ oder der dritte Abschnitt (7) aufweist.

6. Organizer (1) nach einem der vorangegangenen Ansprüche, mit einem einstückig hergestellten Grundkörper (13), welcher den ersten Abschnitt (3), den zweiten Abschnitt (5) und den dritten Abschnitt (7) aufweist.

7. Organizer (1) nach einem der vorangegangenen Ansprüche, welcher mit Blutschlauchsystemelementen bestückt ist.

8. Organizer (1) nach einem der vorangegangenen Ansprüche, bei welchem die Blutschlauchsystemelemente im zusammengeklappte Zustand des Organizers (1) zwischen dem ersten Abschnitt (3) und dem dritten Abschnitt (7) angeordnet sind.

9. Organizer (1) nach einem der vorangegangenen Ansprüche, welcher wenigstens ein Polystyrol aufweist oder hieraus, insbesondere mittels Thermoformen, gefertigt ist.

## Claims

1. An organizer (1) comprising at least one first portion, one second portion, and one third portion, wherein the first portion (3) is connected to the second portion (5) so as to be movable relatively to the second portion (5), and wherein the second portion (5) is connected to the third portion (7) so as to be movable relatively to the third portion (7), wherein at least one dedicated folding area (9) is configured between the first portion (3) and the second portion (5) and/or between the second portion (5) and the third portion (7), wherein the dedicated folding area (9) has a material thickness which is less than a material thickness of the first portion (3) and/or of the second portion (5) and/or of the third portion (7), **characterized in that** the second portion (5) and at least one of the first portion (3) and the third portion (7) each comprise at least one retainer element (11) for reversibly accommodating blood tube system elements or blood tube components.

2. The organizer (1) according to claim 1, wherein the first portion (3) and/ or the third portion (7) are connected to the second portion (5) by at least one film hinge.

3. The organizer (1) according to any one of the preceding claims, wherein the third portion (7) is connected to the first portion (3) via the second portion (5) only.

4. The organizer (1) according to any one of the preceding claims, wherein the second portion (5) is configured in several parts.

5. The organizer (1) according to any one of the preceding claims, wherein the second portion (5) has a more rigid structure than the first portion (3) and/or the third portion (7).

6. The organizer (1) according to any one of the preceding claims, comprising an integrally manufactured base body (13) which includes the first portion (3), the second portion (5) and the third portion (7).

7. The organizer (1) according to any one of the preceding claims, which is equipped with blood tube system elements.

8. The organizer (1) according to any one of the preceding claims, wherein the blood tube system elements are disposed between the first portion (3) and the third portion (7) in the folded state of the organizer (1).

9. The organizer (1) according to any one of the preceding claims, which comprises at least one polystyrene or is made thereof, in particular by means of thermoforming.

## Revendications

1. Organiseur (1) avec au moins une première section, une seconde section et une troisième section, où la première section (3) est connectée avec la seconde section (5) de façon à être mobile par rapport à la seconde section (5) et où la seconde section (5) est connectée avec la troisième section (7) de façon à être mobile par rapport à la troisième section (7), dans lequel est formée au moins une zone prévue de pliage (9) entre la première section (3) et la troisième section (5) et / ou entre la seconde section (5) et la troisième section (7), dans lequel la zone prévue de pliage (9) présente une épaisseur de matériau plus petite que l'épaisseur de matériau de la première section (3) et / ou de la seconde section (5) et / ou de la troisième section (7), **caractérisé en ce que** la seconde section (5) ainsi que la première section (3) et / ou la troisième section (7) comprennent, chacun, au moins un élément de maintien (11) pour recevoir des éléments de système à tubes sanguins ou des parties de tubes sanguins de manière réversible.

2. Organiseur (1) selon la première revendication, dans lequel la première section (3) et / ou la troisième section (7) sont connectées avec la seconde section (5) au moyen d'au moins un film formant charnière.

3. Organiseur (1) selon l'une quelconque des revendications précédentes, dans lequel la troisième section (7) est connectée avec la première section (3) uniquement par le biais de la seconde section (5).

4. Organiseur (1) selon l'une quelconque des revendications précédentes, dans lequel la seconde section (5) est formée de plusieurs parties.

5. Organiseur (1) selon l'une quelconque des revendications précédentes, dans lequel la seconde section (5) présente une structure plus rigide que la première section (3) et / ou que la troisième section (7).

6. Organiseur (1) selon l'une quelconque des revendications précédentes avec un corps de base (13) fabriqué d'une seule pièce présentant la première section (3), la seconde section (5) et la troisième section (7).

7. Organiseur (1) selon l'une quelconque des revendications précédentes équipé d'éléments de système à tubes sanguins.

8. Organiseur (1) selon l'une quelconque des revendications précédentes, dans lequel les éléments de système à tubes sanguins sont agencés entre la première section (3) et la troisième section (7) lorsque l'organiseur (1) est dans un état replié.

9. Organiseur (1) selon l'une quelconque des revendications précédentes présentant au moins un polystyrène ou étant fabriqué à partir d'au moins un polystyrène, en particulier par thermoformage.
